(19) Europäisches Patentamt
European Patent Office
Office européen des brevets

(11) Veröffentlichungsnummer: **0 330 132 B1**

# (12) EUROPÄISCHE PATENTSCHRIFT

(45) Veröffentlichungstag der Patentschrift: **21.04.93**

(51) Int. Cl.5: **C07D 405/06**, C07D 405/14, A01N 43/50, A01N 43/653

(21) Anmeldenummer: **89102948.0**

(22) Anmeldetag: **21.02.89**

Die Akte enthält technische Angaben, die nach dem Eingang der Anmeldung eingereicht wurden und die nicht in dieser Patentschrift enthalten sind.

## (54) Azolylmethylcyloalkyloxirane, ihre Herstellung und Verwendung als Pflanzenschutzmittel.

(30) Priorität: **24.02.88 DE 3805684**

(43) Veröffentlichungstag der Anmeldung:
**30.08.89 Patentblatt 89/35**

(45) Bekanntmachung des Hinweises auf die Patenterteilung:
**21.04.93 Patentblatt 93/16**

(84) Benannte Vertragsstaaten:
**AT BE CH DE ES FR GB GR IT LI NL SE**

(56) Entgegenhaltungen:
**EP-A- 0 089 100**
**EP-A- 0 094 564**
**EP-A- 0 196 038**

(73) Patentinhaber: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Karbach, Stefan, Dr.**
**Grundwiesenweg 44**
**W-6730 Neustadt(DE)**
Erfinder: **Kuekenhoehner, Thomas, Dr.**
**Seidelstrasse 2**
**W-6710 Frankenthal(DE)**
Erfinder: **Sauter, Hubert, Dr.**
**Neckarpromenade 20**
**W-6800 Mannheim 1(DE)**
Erfinder: **Lorenz, Gisela, Dr.**
**Erlenweg 13**
**W-6730 Neustadt(DE)**
Erfinder: **Ammermann, Eberhard, Dr.**
**Sachsenstrasse 3**
**W-6700 Ludwigshafen(DE)**

**EP 0 330 132 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolverbindungen, Verfahren zu ihrer Herstellung und diese enthaltende Fungizide, sowie Verfahren zur Bekämpfung von Pilzen.

Es ist bekannt, Cis-2-(1,2,4-Triazol-1-yl-methyl)-2-(tert.-butyl)-3-(4-chlorphenyl)-oxiran als Fungizid zu verwenden (DE-32 18 130.2). Seine Wirkung ist jedoch nicht befriedigend.

Es wurde nun gefunden, daß Azolylmethyloxirane der Formel I

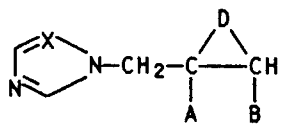

in welcher A und B gleich oder verschieden sind und $C_3$-$C_{12}$-Cycloalkyl, Dioxanyl, Tetrahydropyranyl, Tetrahydrofuranyl, Norbornyl, $C_5$-$C_8$-Cycloalkenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4-C-Atomen substituiert sein können, mit der Maßgabe, daß A und B nicht gleichzeitig Phenyl bedeuten, D den Rest 0 bedeutet, X CH oder N bedeutet sowie deren für Pflanzen verträgliche Säureadditionssalze oder Metallkomplexe eine bessere fungizide Wirkung besitzen als die bekannte Azolverbindung.

Die Verbindungen der Formel I enthalten chirale Zentren und werden im allgemeinen in Form von Racematen bzw. als Diastereomerengemische von erythro- bzw. threo-Formen enthalten. Die erythro- bzw. threo-Diastereomeren lassen sich in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Die vorliegende Erfindung umfaßt sowohl die einheitlichen Diastereomere bzw. Enantiomere wie auch deren bei der Synthese anfallende Gemische.

A bzw. B bedeuten beispielsweise Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl, 2-Methoxyphenyl, 3-Methoxyphenyl, 4-Methoxy phenyl, 2,4-Dimethoxyphenyl, Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluormethylphenyl, 3-Trifluormethylphenyl, 4-Trifluormethylphenyl, Cyclopropyl, Cyclobutyl, Cyclopentyl, Cyclohexyl, Cyclooctyl, Cyclododecyl, Tetrahydropyranyl, Tetrahydrofuranyl, 2-Cyclohexenyl, 3-Cyclohexenyl, Norbornyl, 1,3-Dioxan-2-yl, 1,4-Dioxan-2-yl.

Säureadditionssalze sind beispielsweise die Hydrochloride, Bromide, Sulfate, Nitrate, Phosphate, Oxalate, oder Dodecylbenzolsulfonate. Die Wirksamkeit der Salze geht auf das Kation zurück, so daß das Anion i.a. beliebig ist. Die erfindungsgemäßen Wirkstoffsalze werden hergestellt durch Umsetzung der Azolylmethyloxirane mit den Säuren.

Die Metallkomplexe der Wirkstoffe oder ihre Salze können z.B. mit Kupfer, Zink, Zinn, Mangan, Eisen, Kobalt oder Nickel gebildet werden, indem man die Azolylmethyloxirane mit entsprechenden Metallsalzen umsetzt, z.B. mit Kupfersulfat, Zinkchlorid, Zinnchlorid, Mangansulfat, Eisenchlorid, Kobaltsulfat, Nickelsulfat.

Die Verbindungen der Formel I können z.B. hergestellt werden, indem man

a) eine Verbindung der Formel II

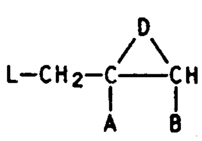

II,

in welcher A, B und D die oben angegebenen Bedeutungen haben und L eine nucleophil substituierbare Abgangsgruppe (z.B. Halogen, OH) bedeutet, mit einer Verbindung der Formel III

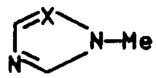

III,

in der Me ein Wasserstoffatom oder ein Metallatom (z.B. Na, K) bedeutet und X die oben angegebene Bedeutung hat, zur Umsetzung bringt, oder

b) eine Verbindung der Formel IV

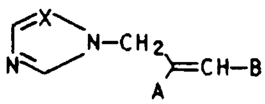

IV,

in welcher A, B und X die oben angegebene Bedeutung haben, in die Epoxide überführt.

Die Reaktion a) erfolgt - falls Me ein Wasserstoffatom bedeutet - gegebenenfalls in Gegenwart eines Lösungs- oder Verdünnungsmittels, gegebenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essisäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Cäsiumcarbonat oder Natrium-, Kalium- oder Cäsiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quaternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-6, Dibenzo-18-krone-6 oder Dicyclohexano-18-krone-6 in Frage.

Die Umsetzung wird im allgemeinen bei Temperaturen zwischen 20 und 150 °C, drucklos oder unter Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120 °C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert-butoxid, Lithium, Natrium- oder Kalium-triphenylmethyl und Naphthalinlithium, -natrium oder kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone z.B. Aceton, Ether, z.B. Diethylether, Tetrahydrofuran und insbesondere Chlorkohlenwasserstoffe, z.B. Methylenchlorid oder Chloroform, in Frage.

Man arbeitet im allgemeinen zwischen 0 und 100 °C, vorzugsweise bei 20 bis 80 °C. Bei Anwesenheit eines Lösungsmittels wird zweckmäßigerweise beim Siedepunkt des jeweiligen Lösungsmittels gearbeitet.

Die neuen Ausgangsverbindungen II erhält man durch Epoxidierung der entsprechenden Olefine V:

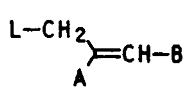

V,

(vgl. G. Dittus in Houben-Weyl-Müller, Methoden der Organischen Chemie, Georg Thieme Verlag, Stuttgart, 1965, Bd. VI, 3, Seite 385ff).

Die Verbindung V stellt man her, indem man Olefine der Formel VI

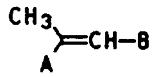 VI,

nach den bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100 ° C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100 ° C.

Die so erhaltenen Allylhalogenide bzw. -alkohole V werden anschließend in die entsprechenden Epoxide II (L = Halogen, OH) übergeführt. Dazu oxidiert man die Olefine V mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat. Man arbeitet zwischen 10 und 100 ° C und katalysiert die Reaktion gegebenenfalls z.B. mit Iod, Natriumwolframat oder Licht. Zur Oxidation eignen sich auch alkalische Lösungen von Wasserstoffperoxid (ca. 30%ig) in Methanol, Ethanol, Aceton oder Acetonitril bei 25 bis 30 ° C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Während die so erhaltenen Epoxihalogenide II (L = Halogen) gemäß Verfahren a) sofort umgesetzt werden können, überführt man die entsprechenden Epoxialkohole II (L = OH) in reaktive Ester, die dann mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b) herstellen.

Die Verbindungen der Formel IV werden z.B. erhalten, indem man eine Verbindung der Formel V mit einer Verbindung der Formel III z.B. in einem Lösungsmittel umsetzt.

Die folgenden Beispiele erläutern die Herstellung der Wirkstoffe.

I. Herstellung der Ausgangsstoffe

Vorschrift A

Zu einer Lösung von 42 g 2,4-Dichlorbenzaldehyd in 200 ml Methanol werden 3,9 g Natriumhydroxid in 30 ml Wasser gegeben. Das Reaktionsgemisch wird auf 10 ° C gekühlt und schnell 30,7 g Tetrahydropyranylacetaldehyd zugetropft, wobei die Temperatur in der Lösung auf 30 ° C ansteigt. Nach zweistündigem Rühren bei Raumtemperatur wird der farblosen Reaktionslösung 200 ml Wasser zugesetzt und die entstandene Emulsion mit Methyl-tert.-butylether ausgeschüttelt. Die organische Phase wird abgetrennt, über Natriumsulfat getrocknet und im Vakuum eingedampft. Man erhält aus Methyl-tert.-butylether/n-Hexan 43 g (63%) E/Z-2-(tetrahydropyran-4-yl)-3-(2,4-dichlorphenyl)-propenal mit dem Schmelzpunkt 82 - 85 ° C.

Vorschrift B

21,3 g E/Z-2-(Tetrahydropyran-4-yl)-3-(2,4-dichlorphenyl)-propenal werden in 200 ml Methanol gelöst und 0,85 ml Natronlauge (konz.) zugesetzt. Die Reaktionslösung wird bei 0°C gerührt, während 5,5 g Wasserstoffperoxid (ca. 50 Gew.%) langsam zugetropft werden, wobei die Innentemperatur von 30°C nicht überschritten wird. Nach beeendeter Zugabe wird sechs Stunden bei Raumtemperatur gerührt und anschließend 0,91 g Natriumborhydrid zugegeben, das in wenig 10 %iger Natronlauge gelöst war. Nachdem das Reaktionsgemisch 18 Stunden bei Raumtemperatur rührte, wird der Lösung 100 ml Wasser zugesetzt und die entstandene Emulsion mit Methlyenchlorid ausgeschüttelt. Die organische Phase wird über Natriumsulfat getrocknet, eingeengt und der Rückstand aus Isopropanol umkristallisiert. Man erhält 19,5 g (86%) cis-2-Hydroxymethyl-2-(tetrahydropyran-4-yl)-3-(2,4-dichlorphenyl)-oxiran, Fp.: 89°C.

Vorschrift C

Zu einer Lösung von 19,5 g cis-2-Hydroxymethyl-2-(tetrahydropyran-4-yl)-3-(2,4-dichlorphenyl)-oxiran, in 100 ml Methylenchlorid und 20 g Triethylamin werden bei Raumtemperatur 14,1 g 4-Methylbenzolsulfonsäurechlorid zugesetzt. Nach 24 Stunden wird das Reaktionsgemisch mit wäßriger Natriumhydrogencarbonat-Lösung und Wasser gewaschen, über Natriumsulfat getrocknet und im Vakuum eingedampft. Kristallisation des verbleibenden Rückstandes aus Methyl-tert.-butylether/n-Hexan ergeben 26 g (89 %) cis-2-(4-Methylphenylsulfonyloxymethyl)-2-(tetrahydropyran-4-yl)-3-(2,4-dichlorphenyl)-oxiran mit einem Schmelzpunkt von 102 - 104°C.

Vorschrift D

Zu einer Lösung von 40 g 3-Cyclohexencarbaldehyd in 300 ml Methanol werden 5,5 g Natriumhydroxyd in 30 ml Wasser gegeben. Das Reaktionsgemisch wird auf 10°C gekühlt und schnell 49,7 g 4-Fluorphenylacetaldehyd zugetropft, wobei die Temperatur in der Lösung 30°C nicht übersteigt. Nach zweistündigem Rührem bei Raumtemperatur wird der farblosen Reaktionslösung 200 ml Wasser zugesetzt und die entstandene Emulsion mit Methyl-tert.-butylether ausgeschüttelt. Die organische Phase wird abgetrennt, über Natriumsulfat getrocknet und im Vakuum eingedampft. Bei der anschließenden Destillation des verbleibenden Rückstandes werden bei 0,5 mbar und 136 - 142°C Übergangstemperatur 34 g (41 %) Z-2-(4-Fluorphenyl)-3-(3-cyclohexenyl)-propenal erhalten.

Vorschrift E

Eine Lösung von 34 g Z-2-(4-Fluorphenyl)-3-(3-cyclohexenyl)-propenal in 200 ml Methanol wird mit 2 g Natriumborhydrid versetzt, das in wenig 10 %iger Natronlauge gelöst ist. Nachdem das Reaktionsgemisch 18 Stunden bei Raumtemperatur rührte, wird der Lösung 100 ml Wasser zugesetzt und die entstandene Emulsion mit Methylenchlorid ausgeschüttelt. Die isolierte organische Phase wird daraufhin über Natriumsulfat getrocknet und eingeengt. Man erhält 34 g (99 %) Z-1-Hydroxymethyl-1-(4-fluorphenyl)-2-(3-cyclohexenyl)-ethen.

Vorschrift F

Zu einer Lösung von 34 g Z-1-Hydroxymethyl-1-(4-fluorphenyl)-2-(3-cyclohexenyl)-ethen in 200 ml Methylenchlorid und 29 g Triethylamin werden bei Raumtemperatur 32,5 g 4-Methylbenzolsulfonsäurechlorid zugesetzt. Nach 24 Stunden wird das Reaktionsgemisch mit wäßriger Natriumhydrogencarbonat-Lösungund Wasser gewaschen, über Natriumsulfat getrocknet und im Vakuum eingedampft. Aus dem Rückstand erhielt man 49,7 g (95 %) Z-1-(4-Methylphenylsulfonyloxymethyl)-1-(4-fluorphenyl)-2-(3-cyclohexenyl)-ethen.

II. Herstellung der Endprodukte

Beispiel 1

Eine Lösung von 4,9 g 1,2,4-Triazol in 100 ml N-Methyl-pyrrolidon wird mit 2,7 g Natriumhydroxid versetzt und für 30 Minuten auf 50°C erwärmt. Nachdem das Reaktionsgemisch auf Raumtemperatur gekühlt wurde, wird der Lösung 26 g cis-2-(4-Methylphenylsulfonyloxymethyl)-2-(tetrahydropyran-4-yl)-3-(2,4-dichlorphenyl)-oxiran, das in 100 ml N-Methyl-pyrrolidon gelöst ist, langsam zugetropft und 12 Stunden bei Raumtemperatur gerührt. Anschließend wird 200 ml Wasser zugegeben und mehrmals mit Methyl-tert.-butylether extrahiert; die organische Phase mit Wasser gewaschen, über Natriumsulfat getrocknet und im Vakuum eingeengt. Man erhält aus Methyl-tert.-butyl-ether/n-Hexan 14 g cis-2-(1,2,4-Triazol-1-yl-methyl)-2-(tetrahydropyran-4-yl)-3-(2,4-dichlor-phenyl)-oxiran mit dem Schmelzpunkt 155 - 158 ° C (Verbindung Nr. 1).

Entsprechend Beispiel 1 können die in der Tabelle 1 aufgeführten Verbindungen hergestellt werden.

Tabelle 1

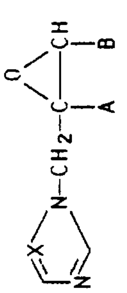

| Nr. | A | B | X | Schmp./IR | Isomer |
|---|---|---|---|---|---|
| 1 | 4-tetrahydropyranyl | 2,4-$Cl_2$-$C_6H_3$ | N | 155-158 °C | cis |
| 2 | 4-tetrahydropyranyl | 2-Cl-$C_6H_4$ | N | 101-103 °C | cis |
| 3 | 4-tetrahydropyranyl | 4-Cl-$C_6H_4$ | N | 157-163 °C | cis |
| 4 | 4-tetrahydropyranyl | 2-F-$C_6H_4$ | N | 131-134 °C | cis |
| 5 | 4-F-$C_6H_4$ | Cyclopropyl | N | 105-108 °C | cis/trans 35:65 |
| 6 | 4-F-$C_6H_4$ | Cyclopentyl | N | 50- 56 °C | cis/trans 60:40 |
| 7 | 4-F-$C_6H_4$ | Cyclohexyl | N | Harz | cis |
| 8 | 4-F-$C_6H_4$ | Cyclooctyl | N | - | - |
| 9 | 4-F-$C_6H_4$ | Cyclododecyl | N | - | - |
| 10 | 2-Cl-$C_6H_4$ | Cyclopropyl | N | - | - |
| 11 | 2-Cl-$C_6H_4$ | Cyclopentyl | N | - | - |
| 12 | 2-Cl-$C_6H_4$ | Cyclohexyl | N | - | - |
| 13 | 4-Cl-$C_6H_4$ | Cyclopropyl | N | - | - |
| 14 | 4-Cl-$C_6H_4$ | Cyclopentyl | N | - | - |
| 15 | 4-Cl-$C_6H_4$ | Cyclohexyl | N | - | - |
| 16 | 2,4-$Cl_2$-$C_6H_3$ | Cyclopropyl | N | - | - |
| 17 | 2,4-$Cl_2$-$C_6H_3$ | Cyclopentyl | N | - | - |
| 18 | 2,4-$Cl_2$-$C_6H_3$ | Cyclohexyl | N | - | - |
| 19 | 4-F-$C_6H_4$ | Norbornyl | N | 212-215 °C | cis/trans 50:50 |
| 20 | 2-F-$C_6H_4$ | Norbornyl | N | - | - |
| 21 | 2-Cl-$C_6H_4$ | Norbornyl | N | - | - |

| Nr. | A | B | X | Schmp./IR | Isomer |
|---|---|---|---|---|---|
| 22 | $4-Cl-C_6H_4$ | Norbornyl | N | - | - |
| 23 | $2,4-Cl_2-C_6H_3$ | Norbornyl | N | - | - |
| 24 | $2-Cl-C_6H_4$ | 3-Cyclohexenyl | N | - | - |
| 25 | $4-Cl-C_6H_4$ | 3-Cyclohexenyl | N | - | - |
| 26 | $2,4-Cl_2-C_6H_3$ | 3-Cyclohexenyl | N | - | - |
| 27 | $2-F-C_6H_4$ | 3-Cyclohexenyl | N | - | |
| 28 | $4-F-C_6H_4$ | 3-Cyclohexenyl | N | 1510,1273,1139 840 $cm^{-1}$ | cis/trans 60:40 |
| 29 | $4-F-C_6H_4$ | 2-Cyclohexenyl | N | - | - |
| 30 | $2-F-C_6H_4$ | 2-Cyclohexenyl | N | - | - |
| 31 | $2-Cl-C_6H_4$ | 2-Cyclohexenyl | N | - | - |
| 32 | $4-Cl-C_6H_4$ | 2-Cyclohexenyl | N | - | - |
| 33 | $2,4-Cl_2-C_6H_3$ | 2-Cyclohexenyl | N | - | - |
| 34 | $2,4-Cl_2-C_6H_3$ | 3-Cyclopentenyl | N | - | - |
| 35 | $2-Cl-C_6H_4$ | 3-Cyclopentenyl | N | - | - |
| 36 | Cyclohexyl | $2-Cl-C_6H_4$ | N | 106-108°C | cis |
| 37 | Cyclohexyl | $4-Cl-C_6H_4$ | N | Harz | cis/trans 50:50 |
| 38 | Cyclohexyl | $2,4-Cl_2-C_6H_3$ | N | 109-111°C | cis |
| 39 | Cyclohexyl | $2-F-C_6H_4$ | N | 90- 92°C | cis |
| 40 | Cyclohexyl | $4-F-C_6H_4$ | N | 2928,2854,1511 1225,1020 $cm^{-1}$ | cis |
| 41 | Cyclopentyl | $2-Cl-C_6H_4$ | N | - | - |
| 42 | Cyclopentyl | $4-Cl-C_6H_4$ | N | - | - |
| 43 | Cyclopentyl | $2-OCH_3-C_6H_4$ | N | - | - |
| 44 | Cyclohexyl | Cyclohexyl | N | - | - |
| 45 | Cyclohexyl | Cyclopentyl | N | - | - |
| 46 | Cyclopropyl | $2,4-Cl_2-C_6H_3$ | N | - | - |
| 47 | Cyclopropyl | $2-Cl-C_6H_4$ | N | - | - |
| 48 | Cyclopropyl | $4-Cl-C_6H_4$ | N | - | - |

| Nr. | A | B | X | Schmp./IR | Isomer |
|---|---|---|---|---|---|
| 49 | Cyclopropyl | 4-F-$C_6H_4$ | N | - | - |
| 50 | Cyclopropyl | 2-F-$C_6H_4$ | N | - | - |
| 51 | 3-Cyclohexenyl | 2-Cl-$C_6H_4$ | N | - | - |
| 52 | 3-Cyclohexenyl | 4-Cl-$C_6H_4$ | N | - | - |
| 53 | 3-Cyclohexenyl | 2,4-$Cl_2$-$C_6H_3$ | N | - | - |
| 54 | 3-Cyclohexenyl | 2-F-$C_6H_4$ | N | - | - |
| 55 | 3-Cyclohexenyl | 4-F-$C_6H_4$ | N | - | - |
| 56 | 2-Cyclohexenyl | 2-Cl-$C_6H_4$ | N | - | - |
| 57 | 2-Cyclohexenyl | 4-Cl-$C_6H_4$ | N | - | - |
| 58 | 2-Cyclohexenyl | 2-F-$C_6H_4$ | N | - | - |
| 59 | 2-Cyclohexenyl | 4-F-$C_6H_4$ | N | - | - |
| 60 | 2-tetrahydrofuranyl | 2-Cl-$C_6H_4$ | N | - | - |
| 61 | 2-tetrahydrofuranyl | 4-Cl-$C_6H_4$ | N | - | - |
| 62 | 2-tetrahydrofuranyl | 2,4-$Cl_2$-$C_6H_3$ | N | - | - |
| 63 | 2-tetrahydrofuranyl | 2-F-$C_6H_4$ | N | - | - |
| 64 | 2-tetryhydrofuranyl | 4-F-$C_6H_4$ | N | - | - |
| 65 | 4-F-$C_6H_4$ | 2-tetrahydrofuranyl | N | - | - |
| 66 | 2-Cl-$C_6H_4$ | 2-tetrahydrofuranyl | N | - | - |
| 67 | 4-Cl-$C_6H_4$ | 2-tetrahydrofuranyl | N | - | - |
| 68 | 2,4-$Cl_2$-$C_6H_3$ | 2-tetrahydrofuranyl | N | - | - |
| 69 | 2-F-$C_6H_4$ | 2-tetrahydrofuranyl | N | - | - |
| 70 | Cyclohexyl | 2,4-$Cl_2$-$C_6H_3$ | CH | 72 - 74°C | cis |
| 71 | Cyclohexyl | 4-F-$C_6H_4$ | CH | Harz | cis/trans 50:50 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie

Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara Viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine oder Amide (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 6 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 7 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 19 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 28 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 36 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 6 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 7 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde cis-2-(1,2,4-Triazol-1-yl-methyl)-2-(tert.butyl)-3-(4-chlorphenyl)-oxiran (A) - bekannt aus DE 3 218 130.2 - benutzt.

Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22 °C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Abtrocknen des Spritzbelages wurden die Versuchspflanzenim Gewächshaus bei Temperaturen zwischen 20 und 22 °C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 2, 6, 7 und 19 bei der Anwendung als 0,025 %ige ( Gew.%) Spritzbrühen eine bessere fungizide Wirkung zeigen (97 %) als der bekannte Vergleichswirkstoff A (60 %).

Anwendungsbeispiel 2

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 - 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 28 und 36 bei der Anwendung als 0,05 %ige Spritzbrühe eine bessere fungizide Wirkung (90 %) zeigen als der bekannte Vergleichswirkstoff A (60 %).

Anwendungsbeispiel 3

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 - 22°C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 6, 7, 28 und 36 bei der Anwendung als 0,05 %ige Spritzbrühe eine gute fungizide Wirkung (97 %) zeigen.

**Patentansprüche**

**1.** Azolylmethyloxirane der allgemeinen Formel I

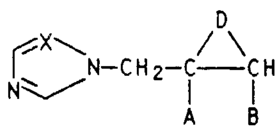

I,

in welcher A und B gleich oder verschieden sind und $C_3$-$C_{12}$-Cycloalkyl, Dioxanyl, Tetrahydropyranyl, Tetrahydrofuranyl, Norbornyl, $C_5$-$C_8$-Cycloalkenyl oder Phenyl bedeuten, wobei diese Reste durch Halogen, Nitro, Phenoxy, Amino, Alkyl, Alkoxy oder Halogenalkyl mit jeweils 1 bis 4-C-Atomen substituiert sein können, mit der Maßgabe, daß A und B nicht gleichzeitig Phenyl bedeuten, D den Rest O bedeutet, X CH oder N bedeutet, sowie deren für Pflanzen verträgliche Säureadditionssalze oder Metallkomplexe.

**2.** Azolylmethyloxirane der allgemeinen Formel I, in denen A einen gegebenenfalls durch Fluor oder Chlor substituierten Phenylrest, D den Rest O bedeutet und B und X die in Anspruch 1 angegebene Bedeutung haben.

**3.** Verfahren zur Herstellung der Azolylmethyloxirane der Formel I gemäß Anspruch 1, in der D den Rest O bedeutet, dadurch gekennzeichnet, daß man

a) eine Verbindung der Formel II

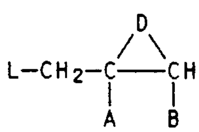

II,

in welcher A, B und D die oben angegebenen Bedeutungen haben und L eine nucleophil substituierbare Abgangsgruppe darstellt, mit einer Verbindung der Formel III

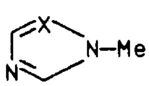

III,

in der Me ein Wasserstoffatom oder ein Metallatom bedeutet und X die oben angegebene Bedeutung hat, umsetzt oder

b) eine Verbindung der Formel IV

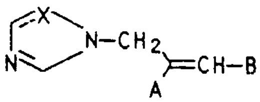

IV,

in welcher A, B und X die oben angegebene Bedeutung haben, in das entsprechende Oxiran überführt und die so erhaltenen Verbindungen gegebenenfalls in ihre Salze mit für Pflanzen verträglichen Säuren überführt.

**4.** Fungizides Mittel, enthaltend einen Trägerstoff und ein Azolylmethyloxiran der Formel I

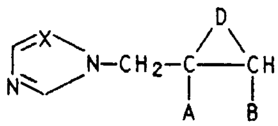

in welcher A und B gleich oder verschieden sind und $C_3$-$C_{12}$-Cycloalkyl, Dioxanyl, Tetrahydropyranyl, Tetrahydrofuranyl, Norbornyl, $C_5$-$C_8$-Cycloalkenyl oder Phenyl bedeuten, wobei diese Reste durch Halogen, Nitro, Phenoxy, Amino, Alkyl, Alkoxy oder Halogenalkyl mit jeweils 1 bis 4-C-Atomen substituiert sein können, mit der Maßgabe, daß A und B nicht gleichzeitig Phenyl bedeuten, D den Rest O bedeutet, X CH oder N bedeutet oder dessen für Pflanzen verträgliches Säureadditionssalz oder Metallkomplex.

**5.** Verfahren zur Bekämpfung von Pilzen, dadurch gekennzeichnet, daß man eine fungizid wirksame Menge eines Azolylmethyloxirans der Formel I

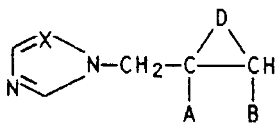

in welcher A und B gleich oder verschieden sind und $C_3$-$C_{12}$-Cycloalkyl, Dioxanyl, Tetrahydropyranyl, Tetrahydrofuranyl, Norbornyl, $C_5$-$C_8$-Cycloalkenyl oder Phenyl bedeuten, wobei diese Reste durch Halogen, Nitro, Phenoxy, Amino, Alkyl, Alkoxy oder Halogenalkyl mit jeweils 1 bis 4-C-Atomen substituiert sein können, mit der Maßgabe, daß A und B nicht gleichzeitig Phenyl bedeuten, D den Rest 0 bedeutet, X CH oder N bedeutet, oder dessen für Pflanzen verträgliches Säureadditionssalz oder Metallkomplex auf die Pilze oder auf durch Pilzbefall bedrohte Materialien, Flächen, Pflanzen oder Saatgüter einwirken läßt.

**6.** Verbindung gemäß Anspruch 1, dadurch gekennzeichnet, daß
A 4-Fluorphenyl,
B Cyclopentyl,
D den Rest O und
X den Rest N
bedeuten.

**7.** Verbindung gemäß Anspruch 1, dadurch gekennzeichnet, daß A 4-Fluorphenyl, B Cyclohexyl, D den Rest O und X den Rest N bedeuten.

## Claims

1. An azolylmethyloxirane of the general formula I

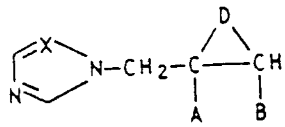

I,

where A and B are identical or different and each is $C_3$-$C_{12}$-cycloalkyl, dioxanyl, tetrahydropyranyl, tetrahydrofuranyl, norbornyl, $C_5$-$C_8$-cycloalkenyl or phenyl, it being possible for each of these radicals to be substituted by halogen, nitro, phenoxy, amino, alkyl, alkoxy or haloalkyl, each of 1 to 4 carbon atoms, with the proviso that A and B are not simultaneously phenyl, D is O, X is CH or N, and its plant-tolerated acid addition salts or metal complexes.

2. An azolylmethyloxirane of the general formula I, where A is unsubstituted or fluoro- or chloro-substituted phenyl, D is O, and B and X have the meanings given in claim 1.

3. A process for the preparation of the azolylmethyloxiranes of the formula I as claimed in claim 1, in which D is O, wherein

a) a compound of the formula II

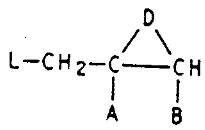

II,

where A, B and D have the above meanings and L is a nucleophilically substitutable leaving group, is reacted with a compound of the formula III

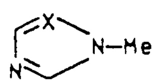

III,

where Me is a hydrogen atom or a metal atom and X has the above meanings, or

b) a compound of the formula IV

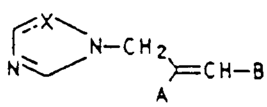

IV,

where A, B and X have the above meanings, is converted into the corresponding oxirane and the compounds thus obtained are, if desired, converted into their salts with plant-tolerated acids.

4. A fungicidal agent containing a carrier and an azolylmethyloxirane of the formula I

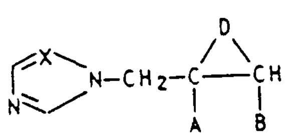

where A and B are identical or different and each is $C_3$-$C_{12}$-cycloalkyl, dioxanyl, tetrahydropyranyl,

tetrahydrofuranyl, norbornyl, $C_5$-$C_8$-cycloalkenyl or phenyl, it being possible for each of these radicals to be substituted by halogen, nitro, phenoxy, amino, alkyl, alkoxy or haloalkyl, each of 1 to 4 carbon atoms, with the proviso that A and B are not simultaneously phenyl, D is O, X is CH or N, or a plant-tolerated acid addition salt or metal complex thereof.

**5.** A method for controlling fungi, wherein a fungicidally effective amount of an azolylmethyloxirane of the formula I

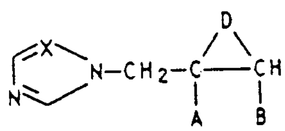

where A and B are identical or different and each is $C_3$-$C_{12}$-cycloalkyl, dioxanyl, tetrahydropyranyl, tetrahydrofuranyl, norbornyl, $C_5$-$C_8$-cycloalkenyl or phenyl, it being possible for each of these radicals to be substituted by halogen, nitro, phenoxy, amino, alkyl, alkoxy or haloalkyl, each of 1 to 4 carbon atoms, with the proviso that A and B are not simultaneously phenyl, D is O, X is CH or N, or a plant-tolerated acid addition salt or metal complex thereof, is allowed to act on the fungi, or the materials, areas, plants or seed threatened by fungus attack.

**6.** A compound as claimed in claim 1, where A is 4-fluorophenyl, B is cyclopentyl, D is O, and X is N.

**7.** A compound as claimed in claim 1, where A is 4-fluorophenyl, B is cyclohexyl, D is O, and X is N.

## Revendications

**1.** Azolylméthyloxirannes de formule générale I

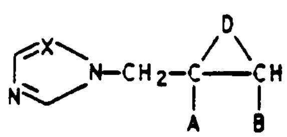

I,

dans laquelle A et B sont identiques ou différents et représentent cycloalkyle en C3-C12, dioxanyle, tétrahydropyranyle, tétrahydrofuranyle, norbornyle, cycloalcényle en C5-C8 ou phényle, ces restes pouvant être substitués par halogène, nitro, phénoxy, amino, alkyle, alcoxy ou halogénalkyle à 1 à 4 atomes C dans chaque cas, étant entendu que A et B ne représentent pas simultanément phényle, D représente le reste O, X est CH ou N, ainsi que leurs sels d'addition d'acide ou complexes métalliques tolérés par les plantes.

**2.** Azolylméthyloxirannes de formule générale I, dans lesquels A représente un reste phényle éventuellement substitué par fluor ou chlore, D représente le reste O et B et X ont les significations données dans la revendication 1.

**3.** Procédé de préparation des azolylméthyloxirannes de formule I, selon la revendication 1, dans laquelle D représente le reste O, caractérisé par le fait que

a) on fait réagir un composé de formule II

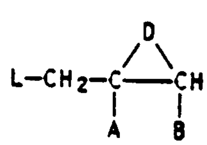

II,

dans laquelle A, B et D ont les significations données plus haut et L représente un groupe éliminable

pouvant être substitué par un réactif nucléophile, avec un composé de formule III

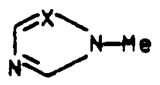 III.

dans laquelle Me représente un atome d'hydrogène ou un atome de métal et X a la signification donnée plus haut, ou
b) on transforme un composé de formule IV

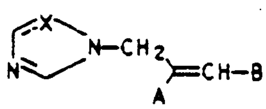 IV.

dans laquelle A, B et X ont les significations données plus haut, en l'oxiranne correspondant et on transforme éventuellement les composés ainsi obtenus en leurs sels avec des acides tolérés par les plantes.

**4.** Agent fongicide contenant un véhicule et un azolylméthyloxiranne de formule I

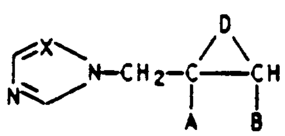

dans laquelle A et B sont identiques ou différents et représentent cycloalkyle en C3-C12, dioxanyle, tétrahydropyranyle, tétrahydrofuranyle, norbornyle, cycloalcényle en C5-C8 ou phényle, ces restes pouvant être substitués par halogène, nitro, phénoxy, amino, alkyle, alcoxy ou halogénalkyle à 1 à 4 atomes C dans chaque cas, étant entendu que A et B ne représentent pas simultanément phényle, D représente le reste O, X est CH ou N, ou son sel d'addition ou complexe métallique tolérés par les plantes.

**5.** Procédé de lutte contre les champignons, caractérisé par le fait que l'on fait agir une quantité efficace au point de vue fongicide d'un azolylméthyloxiranne de formule I

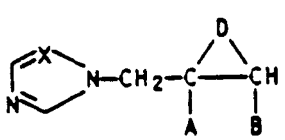

dans laquelle A et B sont identiques ou différents et représentent cycloalkyle en C3-C12, dioxanyle, tétrahydropyranyle, tétrahydrofuranyle, norbornyle, cycloalcényle en C5-C8 ou phényle, ces restes pouvant être substitués par halogène, nitro, phénoxy, amino, alkyle, alcoxy ou halogénalkyle à 1 à 4 atomes C dans chaque cas, étant entendu que A et B ne représentent pas simultanément phényle, D représente le reste O, X est CH ou N, ou son sel d'addition d'acide ou complexe métallique tolérés par les plantes, sur les champignons ou les matériaux, surfaces, plantes ou semences menacés par l'attaque des champignons.

**6.** Composé selon la revendication 1, caractérisé par le fait que
A = 4-fluorophényle,
B = cyclopentyle,

D = le reste O et
X = le reste N.

7. Composé selon la revendication 1, caractérisé par le fait que A représente 4-fluorophényle, B cyclohexyle, D le reste O et X le reste N.